Europäisches Patentamt

European Patent Office

Office européen des brevets

(19)

(11) EP 0 712 426 B1

# (12) FASCICULE DE BREVET EUROPEEN

(45) Date de publication et mention
de la délivrance du brevet:
**04.06.1997 Bulletin 1997/23**

(21) Numéro de dépôt: **94922905.8**

(22) Date de dépôt: **11.07.1994**

(51) Int Cl.6: **C08K 9/10**, G01N 33/58,
C08F 2/44

(86) Numéro de dépôt international:
**PCT/FR94/00867**

**WO 95/02635 (26.01.1995 Gazette 1995/05)**

(54) **LATEX FLUORESCENT COMPORTANT AU MOINS DEUX FLUOROCHROMES, PROCEDE D'OBTENTION ET APPLICATION**

FLUORESZENZLATEX, ENTHALTEND MINDESTENS ZWEI FLUOROCHROME, HERSTELLUNGSVERFAHREN UND ANWENDUNG

FLUORESCENT LATEX COMPRISING AT LEAST TWO FLUOROCHROMES, METHOD FOR PRODUCING SAME, AND USE THEREOF

(84) Etats contractants désignés:
**BE CH DE DK ES FR GB IE IT LI NL SE**

(30) Priorité: **12.07.1993 FR 9308573**

(43) Date de publication de la demande:
**22.05.1996 Bulletin 1996/21**

(73) Titulaire: **SOCIETE PROLABO**
**94120 Fontenay-sous-Bois (FR)**

(72) Inventeurs:
• **VASLIN, Sophie Parc des Bords de Marne**
**F-94360 Bry-sur-Marme (FR)**
• **RICHARD, Joel Résidence Mermoz**
**F-60500 Chantilly (FR)**

• **TEYCHENNE, Dominique**
**F-93160 Noisy-le-Grand (FR)**
• **RICCHIERO, Frédéric**
**Résidence "Le Domitien B.46"**
**F-34090 Montpellier (FR)**
• **LERNER, Dan Résidence "Eden Parc E"**
**F-34000 Montpellier (FR)**

(74) Mandataire: **Ahner, Francis et al**
**CABINET REGIMBEAU**
**26, avenue Kléber**
**75116 Paris (FR)**

(56) Documents cités:
**EP-A- 0 280 556**      **WO-A-91/10893**

## Description

La présente invention a pour objet un latex fluorescent dont les particules contiennent encapsulés aux moins deux fluorochromes hydrophobes. Elle concerne également un procédé de préparation dudit latex et l'application de celui-ci comme marqueur, notamment en biologie.

Les latex sont des dispersions aqueuses de particules de polymère, dont la taille est généralement comprise entre 0,05 micron et plusieurs microns. Compte tenu de leur aspect particulaire, les latex offrent une grande surface spécifique qui est exploitée avantageusement dans un grand nombre d'applications et en particulier dans les industries du papier, de peinture, bandes magnétiques, enregistrements et en biologie.

En biologie, des techniques de dosages classiques mettent en oeuvre des latex à titre de support de marqueurs de type enzymes ou radio-isotopes afin de permettre le dosage quantitatif des espèces présentes dans le milieu à analyser.

Cette technique de dosage par marquage moléculaire à l'aide par exemple d'isotope radioactif, est extrêmement précise, fiable et d'une grande sensibilité de détection. Toutefois, elle présente plusieurs inconvénients liés à la dispersion des sources radio-actives, l'exposition prolongée du personnel, la variation temporelle de l'émission de la source liée à la demi-vie de l'élément et surtout à la nécessité en fin d'analyse d'isoler les réactifs libres des réactifs complexés. L'émission radio-active est en effet totalement insensible à l'environnement du marqueur.

Pour les raisons précitées, la technique d'analyse par fluorescence s'est imposée comme l'alternative la plus efficace pour le remplacement de ces marqueurs classiques.

Les avantages du marquage fluorescent, comparativement au marquage radio-actif, sont multiples. Le risque d'exposition radio-active est nul. Le marquage fluorescent présente une excellente stabilité dans le temps et surtout il fournit une réponse plus affinée en raison de la spécificité de l'émission de fluorescence à certains paramètres d'environnement. Son emploi ne nécessite donc pas la mise en oeuvre de séparations post-analyses.

Néanmoins, la mise en oeuvre pratique de la fluorescence notamment dans les dosages biologiques (immunologie, comptage cellulaire, cytométrie de flux) requiert que certains problèmes soient résolus.

La fluorescence, issue du simple greffage moléculaire, présente une sensibilité insuffisante en vue d'une application pratique généralisée. Il est difficile de la détecter lorsque la concentration des entités présentes dans le milieu est inférieure à $10^{-9}$ mole/litre. Cela limite considérablement l'utilisation de cette technique.

Pour être satisfaisante, la sensibilité de la fluorescence devrait être comparable à celle du marquage radio-actif ce qui implique de pouvoir travailler à des concentrations comprises entre $10^{-12}$ et $10^{-15}$ mole de particules par litre.

Par ailleurs, le milieu dans lequel sont présents les composés à doser peut interférer avec la fluorescence émise par les fluorochromes (par exemple diffusion Rayleigh, Raman de l'eau). Il y a bien d'autres composés présents notamment dans le sérum biologique qui, sous l'effet de l'excitation du rayonnement lumineux, émettent également dans une plage située entre 300 et 500 nanomètres. Il est donc souhaitable que le signal d'analyse de fluorescence se situe au-delà de 500 nanomètres avec le plus large écart spectral entre excitation et émission.

Toutefois, on sait que beaucoup de lasers disponibles commercialement présentent une longueur d'onde qui est précisément comprise entre 300 et 500 nanomètres, par exemple le laser Argon émet à 488 nanomètres et le laser Helium-Cadmium à 440 nanomètres. Il est donc souhaitable de pouvoir choisir un spectre d'excitation selon ces longueurs d'ondes, même si cela provoque l'excitation parasite de l'eau ou d'autres substances biologiques, mais que l'émission du latex soit déplacée vers des longueurs d'ondes plus élevées.

La présente invention a précisément pour objectif de pallier ces inconvénients et/ou de résoudre les problèmes indiqués ci-dessus.

D'autres avantages procurés par la présente invention apparaîtront également au vu de la description qui va suivre.

En premier lieu, l'invention est relative à un latex fluorescent dont les particules contiennent encapsulés au moins un fluorochrome hydrophobe A (accepteur) et au moins un fluorochrome hydrophobe D (donneur), A étant différent de D et l'émission du fluorochrome D permettant sous l'action d'un rayonnement lumineux d'exciter le fluorochrome A.

Par le terme encapsulé, on entend le fait que les fluorochromes sont quasiment absents de la surface des particules. Ils sont pour l'essentiel concentrés au sein des particules de polymères de manière à laisser la surface externe de celles-ci disponible.

Les fluorochromes selon l'invention sont sélectionés de manière à couvrir en émission les quatre grandes zones de spectre lumineux à savoir le bleu (400-500 nm), le vert (500-550 nm), le jaune (550-600 nm) et le rouge (600-750nm).

Outre les propriétés spectrales requises, ces fluorochromes satisfont également aux exigences suivantes :

Il sont hydrophobes de manière à favoriser leur insertion dans les particules de latex et s'affranchir de tout relargage ultérieur.

Ils présentent une affinité pour l'intérieur de la particule, c'est-à-dire sont compatibles chimiquement avec le polymère constituant les particules de latex et, le cas échéant, avec les fonctions chimiques présentes dans ce polymère. Cette compatibilité joue un rôle lors de la synthèse du latex fluorescent correspondant.

Enfin, compte tenu de leur emploi en biologie, ces fluorochromes sont stables chimiquement et photochimiquement.

Parmi les fluorochromes convenant à l'invention, on peut plus particulièrement citer les 9,10 diphényl-anthracène (9,10 DPA), 9,10 bis-phényl-éthynyl-anthracène (9,10 BPEA), 1,8 dichloro 9,10 bis-phényl-éthynyl-anthracène (1,8 $Cl_2$-9,10 BPEA), 5-12 bis-phényl-éthynylnaphtacène (5,12 BPEN) 6,13-bis (phényléthynyl)pentacène, tétrabenzo(de, hi,op,st)pentacène, la Coumarine 153, le Rouge de Nil, le 1,4-Di-[2(5-phényloxazolyl)] benzène ou POPOP, le 1,4-Di-[(2,-(4-méthyl-5-phényloxazolyl)] benzène ou DM-POPOP, la tetraphénylporphyrine ou TPP.

De manière générale, les couples de fluorochromes ou les trios comme on le verra par la suite dans une variante de l'invention sont choisis en fonction de la finalité et des conditions d'utilisation des particules de latex. Succintement, le phénomène de transfert d'énergie électronique fait intervenir un donneur D\*, molécule portée à un état excité par une forme quelconque de rayonnement et un accepteur $A_0$ molécule à état fondamental. Sous certaines conditions, si le niveau énergétique de D\* est supérieur ou égal à celui de A\*, l'énergie initialement acquise par le donneur peut être transmise à la molécule A pour former l'espèce excitée A\*, sans qu'il y ait eu excitation directe de $A_0$.

L'accepteur peut alors émettre en lieu et place du donneur, à sa propre longueur d'onde λ (A). L'émission normale du donneur (D\* → D + hν) est remplacée par:

$$1) \ D^* + A_0 \rightarrow D_0 + A^* \text{ (vitesse } K_{tr})$$

puis

$$2) \ A^* \rightarrow A_0 + h\nu \text{ (émission de A à λ (A)).}$$

Dans le cas particulier des couples ou trios de fluorochromes présentés ici, le transfert est effectué par résonance et fait appel à un processus de couplage électromagnétique à travers l'espace qui ne nécessite aucun contact entre le donneur et l'accepteur. Il nécessite par ailleurs, qu'il y ait concordance des sauts d'énergie (entre état fondamental et état excité) pour le couple donneur-accepteur. Il doit donc exister un recouvrement entre le spectre d'émission du donneur D et le spectre d'excitation de l'accepteur A.

A titre indicatif, on peut citer les couples de fluorochromes suivants :

| D | | A |
|---|---|---|
| 9,10 DPA | → | 9,10 BPEA |
| 1,8 $Cl_2$-9,10 BPEA | → | 5,12-BPEN |
| 1,8 $Cl_2$-9,10 BPEA | → | TPP |
| Coumarine 153 | → | 1,8 $Cl_2$-9,10 BPEA |
| 9,10 DPA | → | Coumarine 153 |
| 1,8 $Cl_2$-9,10 BPEA | → | Rouge de Nil |
| 9,10 BPEA | → | Rouge de Nil |
| POPOP | → | Coumarine 153 |
| 9,10 BPEA | → | TPP |

Les couples formés à partir des fluorochromes précités sont avantageux à plusieurs titres.

Il s'agit de produits commerciaux. Ils sont insolubles dans l'eau et sont chimiquement et photochimiquement stables. Compatibles avec les polymères de latex, ils conduisent, lors de leur incorporation au sein des particules de latex, à des marqueurs possédant une sensibilité inattendue et nettement plus performante que celle des marqueurs classiques.

Les particules de latex contenant lesdits fluorochromes sont classiquement constituées de polymères obtenus par polymérisation de monomères insaturés éthyléniquement. Il s'agit d'un homopolymère ou copolymère contenant des motifs dérivés de monomères vinylaromatiques, éthyléniques, d'acides ou d'esters alcanoïques ou éthyléniques éventuellement fonctionnalisés.

Ce type de polymère est facilement accessible à tout homme de l'art et on se contentera d'en citer quelques uns ci-après, à titre non limitatif. Il peut s'agir de :

- monomères éthyléniques de type isoprène, 1,3 butadiène, chlorure de vinylidène, acrylonitrile,
- monomères vinylaromatiques comme le styrène, le bromostyrène, l'alphaméthylstyrène, l'éthylstyrère, le vinyltoluène, le chlorostyrène ou le chlorométhylstyrène ou le vinylnaphtalène,

- acides, esters ou anhydrides alcanoïques commes les acides acrylique, méthacrylique, acrylates et méthacrylates d'alkyle dont le groupe alkyle possède 3 à 10 atomes de carbone, hydroxyalkylacrylates, les acrylamides, les esters d'acides éthyléniques à 4 ou 5 atomes de carbone ainsi que,
- les monomères difonctionnels tels que le divinylbenzène ou le diacrylate de 2,2-diméthyl-1,3-propylène et/ou d'autres monomères copolymérisables insolubles dans l'eau.

Les monomères peuvent porter des groupements anioniques ou cationiques de type sulfate, sulfonate, phosphonate ou ammonium quaternaire. Il peut également s'agir de groupements susceptibles de réagir, directement ou indirectement, avec des groupements fonctionnels de type amine, par exemple, portés par des molécules biologiques comme les protéines et les enzymes. A titre représentatif de ces groupes fonctionnels, on peut citer les halogènes, les groupements carboxyles, amines, isocyanates, aziridines, aldéhydes, sulfonyles et les fonctions époxy, chlorométhyle.

Les monomères, plus particulièrement utilisés dans le cadre de la présente invention appartiennent à la famille des arylènes et/ou des alkylènes. Il s'agit préférentiellement de composés vinylaromatiques tels que : styrène, alphaméthylstyrène, éthylstyrène, tertio-butylstyrène et vinyltoluène. De préférence, ces monomères sont substitués par un ou plusieurs groupements fonctionnels de type halogène, amine, alcoxy, carboxyle et/ou sulfonyle.

Ces monomères sont utilisés seuls ou en mélange entre eux en toute proportion, ou encore en mélange avec un autre monomère copolymérisable choisi parmi ceux précités.

Les particules de polymères peuvent être obtenues par la mise en oeuvre d'une quelconque technique de polymérisation comme la polymérisation en émulsion classique, en microémulsion, en suspension, en microsuspension, ou le cas échéant par polymérisation en milieu organique. Ces techniques familières à l'homme de l'art ne seront pas rappelées ici.

Les particules, contenant le latex fluorescent selon l'invention, sont hydrophobes et possèdent de préférence une taille généralement comprise entre 0,01 et 20 microns et de préférence inférieure à 5 microns. Elles sont calibrées, monodispersées et présentes dans le latex à raison d'une quantité variant entre 0,05 à 10 % en poids du poids total du latex, préférentiellement entre 0,1 et 1 %.

Selon une première variante avantageuse, le latex fluorescent est caractérisé en ce que l'émission du fluorochrome D permet directement d'exciter le fluorochrome A, le spectre d'émission du fluorochrome D recouvrant partiellement le spectre d'excitation du fluorochrome A.

De préférence, les rapports molaires A:D est pour au moins 10 %, de préférence au moins 50 %, en poids des particules supérieur à 1, avantageusement supérieur à 1,2.

Avantageusement, le rapport A/D est supérieur à 1, de préférence supérieur à 1,2. De façon surprenante, on a en effet trouvé que les particules de latex présentant un tel rapport conduisaient à un phénomène d'amplification de l'intensité du signal d'émission.

Selon une autre variante avantageuse, le latex fluorescent est caractérisé en ce que l'émission du fluorochrome D permet d'exciter indirectement le fluorochrome A, les particules du latex contenant au moins un fluorochrome intermédiaire I dont le spectre d'excitation recouvre partiellement le spectre d'émission de D et le spectre d'émission recouvre partiellement le spectre d'excitation de A.

De préférence, le rapport molaire A:I et I:D seront toujours supérieurs à 1, de préférence $\geq$ 1,2. Les particules de latex présentant un tel rapport conduisent également à une amplification de l'intensité du signal d'émission.

L'utilisation d'un trio permet de déplacer encore plus la longueur d'onde d'émission du latex et par conséquent d'éviter encore plus sûrement toute interférence et par ailleurs évite le recouvrement des spectres d'émission de A et d'excitation de D, ce qui rend la quantification encore plus précise.

Parmi les trios utilisables dans le cadre de la présente invention, on peut citer à titre indicatif :

| D | I | A |
|---|---|---|
| POPOP | Coumarine 153 | 1,8 $Cl_2$-9,10 BPEA |
| 9,10 DPA | 9,10 BPEA | Rouge de Nil |
| Coumarine 153 | 1,8 $Cl_2$-9,10 BPEA | TPP |
| Coumarine 153 | 1,8 $Cl_2$-9,10 BPEA | 5,12-BPEN |

Il est possible avec le latex selon l'invention d'arriver à des seuils de détection très inférieures à $10^{-12}$ mole de particules par litre dans les conditions de mesure de fluorescence décrites ci-après.

Il est possible ainsi d'obtenir des seuils de détection allant de $10^{-14}$ à $10^{-17}$ mole de particules par litre.

Les latex fluorescents selon l'invention peuvent être utilisés dans tous les applications des latex classiques bien connues de l'homme du métier (peinture....).

Les latex fluorescents selon l'invention sont plus particulièrement destinés à intervenir directement ou indirectement dans des analyses biologiques. Ils peuvent par exemple être employés à titre de réactifs dans les tests immunologiques, de scintillateurs, de standards de calibration en cytofluorométrie en flux par exemple ou encore comme marqueurs cellulaires. On réalise dans ce dernier cas une phagocytose des particules d'un latex fluorescent par les cellules à étudier.

L'invention a donc pour objet les applications des latex fluorescents dans le domaine biologique. Ces applications requièrent le plus souvent la fixation préalable d'une espèce immunoréactive au latex.

L'invention a en conséquence pour objet des latex fluorescents fonctionnalisés par au moins une espèce immunoréactive.

Le couplage d'un latex fluorescent à une espèce immunoréactive est effectué par l'intermédiaire d'un ou plusieurs groupement(s) fonctionnel(s), réactif(s) et présent(s) à la surface de ces particules.

La procédure classique pour réaliser ce type de couplage, implique la création d'une liaison covalente avec cette espèce immunoréactive, par simple réaction chimique. Les groupements chimiques, présents à la surface des particules, peuvent, soit réagir directement avec les composés immunoréactifs porteurs de fonctions amine ou sulfhydryle libres ou, indirectement après activation. A titre de groupements fonctionnels, on peut en particulier citer les groupements carboxyle, halogénoalkyle, alkylsulfonyle et vinylsulfonyle. Dans le cas de groupements aldéhydes et époxy, ces derniers peuvent être activés chimiquement au préalable pour conduire à une fonction présentant une actitivé supérieure vis-à-vis des espèces immunoréactives.

Sous le terme espèce immunoréactive, on désigne tout composé chimique ou biologique porteur d'au moins un site susceptible de se complexer à une autre molécule spécifique dite récepteur. A titre d'exemples d'espèces immunoréactives, on peut citer les amines primaires, les acides aminés, les peptides, les protéines, les lipoprotéines ou des microorganismes de type virus ou bactéries par exemple. Il peut également s'agir d'un anticorps ou d'une enzyme. Cette espèce immunoréactive a pour mission essentielle de réagir avec une autre espèce, soit par simple affinité biologique, soit par réaction chimique, en se complexant par l'intermédiaire d'une de ses fonctions à un site récepteur de l'espèce à doser.

L'addition de ces espèces immunoréactives, aux particules de latex fluorescentes, se réalise selon des procédés classiques, en utilisant des réactions connues qui ne seront pas rappelées ici.

Les latex fluorescents fonctionnalisés trouvent une application dans le domaine du dosage immunologique. Les latex fonctionnalisés avec un anticorps déterminé sont ainsi capables de reconnaître les antigènes complémentaires présents dans un fluide biologique tel que le sang, l'urine. Le diagnostic dépend de la reconnaissance ou de la non reconnaissance des antigènes. Ce résultat est quantifiable par l'intensité du signal qui dépend de la concentration en antigènes.

Les latex fluorescents trouvent également une application dans le marquage cellulaire. Dans ce cas, une espèce immunoréactive greffée sur les latex fluorescents réagit avec un ou plusieurs antigènes de surface d'une cellule et permet de détecter sa présence par fluorescence.

On citera par exemple la détection de cellules cancéreuses qui entraînent par exemple l'augmentation du nombre de récepteurs de l'EGF (Epidermal Growth Factor).

Cette technique permet également de détecter l'augmentation du taux d'acide nucléique ou l'augmentation du volume cellulaire.

Les latex selon la présente invention trouvent également une application avantageuse dans la cytofluorométrie de flux qui permet de réaliser des mesures quantitatives sur une multiplicité de cellules, mais qui sont analysées une par une. Pour chaque cellule, il est possible de déterminer son volume, sa taille et en présence de marqueurs fluorescents spécifiques, son taux d'ADN et d'ARN ainsi que la présence d'antigènes membranaires, ou d'autres récepteurs par exemple. Associée à cette capacité d'analyse, la séparation physique et sélective des sous-populations cellulaires en fonction de paramètres déterminés à l'avance par l'utilisateur amplifie l'intérêt de cette technique.

L'invention concerne également un procédé de préparation des latex fluorescents décrits ci-dessus.

Le procédé de préparation est caractérisé en ce que l'on effectue l'une et/ou l'autre des étapes suivantes :

- introduction, en cours de polymérisation du ou des monomère(s) destinés à constituer les particules de latex, d'un ou plusieurs des fluorochromes D, A et éventuellement I en suspension dans une fraction du ou d'un des monomère(s),

- mélange d'un ou plusieurs des fluorochromes D, A et éventuellement I, solubilisés dans un solvant non aqueux, avec une dispersion aqueuse de particules de latex.

Les deux étapes seront successivement effectuées lorsque l'une des deux étapes n'aura pas permis, compte tenu du procédé choisi, d'inclure les différents fluorochromes dans les particules de latex.

Ainsi, lorsque l'on introduit au cours de la polymérisation un seul des fluorochromes, il sera nécessaire dans une seconde étape, de mélanger l'autre fluorochrome dans un solvant non aqueux avec la dispersion aqueuse de particules de latex obtenue après polymérisation.

Dans un autre cas, il sera possible d'introduire les différents fluorochromes dans l'étape de mélange (ou gonflement) du latex.

La première étape est généralement appelée étape de surpolymérisation alors que la seconde est appelée étape de gonflement au solvant.

De façon connue, les particules de latex fluorescents, obtenues à l'issue de l'une et/ou l'autre de ces deux méthodes, sont ensuite caractérisées en terme de taux d'occupation en fluorochromes, de fluorescence résiduelle du surnageant et de sensibilité de détection de l'émission de fluorescence.

Le taux d'occupation maximal des particules de latex en fluorochromes hydrophobes est bien entendu fonction de la nature des fluorochromes, de la technique d'insertion mise en oeuvre, de la nature du polymère constituant les particules et de la taille de ces particules. Ce taux d'occupation peut donc varier considérablement et atteindre des valeurs de plusieurs millions de molécules de fluorochromes par particule de latex.

A titre indicatif, pour une particule d'un diamètre de 0,3 µm, ce taux varie de 10 000 à 400 000, de préférence entre 60 000 et 350 000, molécules de fluorochromes par particule de latex.

L'incorporation des fluorochromes à l'intérieur des particules, selon l'une et/ou l'autres des deux techniques précitées, est avantageuses à deux titres :

Elle permet de s'affranchir complètement de tout phénomène de relargage de l'agent fluorochrome au cours du dosage. Aucun phénomène de fluorescence dite résiduelle n'est noté dans le surnageant. Il s'ensuit une fiabilité accrue de la technique de dosage.

La surface externe des particules de latex demeure disponible pour des couplages chimiques ou biologiques.

Un avantage important de la fluorescence sur d'autres techniques comme l'UV-visible ou l'émission radioactive, est de permettre en outre le dosage de composés inhibiteurs. Cette caractéristique offre aux latex fluorescents selon l'invention des applications pratiques supplémentaires, telles que les dosages de traces de métaux lourds, de l'oxygène... Cette technique fait déjà l'objet d'applications en immunologie. Il s'agit plus particulièrement de la méthode d'immuno-fluorescence inverse.

Les exemples présentés ci-après permettront de mettre en évidence d'autres avantages et caractéristiques de la présente invention sans toutefois en limiter la portée.

Les latex fluorescents, faisant objet des exemples ci-après, ont été caractérisés par leur seuil de détection en émission fluorescente et, leurs particules respectives, par leur taux d'occupation en fluorochromes selon les méthodes suivantes :

A - Mesure du seuil de détection de fluorescence

Elle consiste à déterminer la concentration minimale en particules détectables par le fluorimètre dans des conditions acceptables sur le plan du rapport signal/bruit (>10), par un fluorimètre conventionnel et sans désoxygénation du milieu. Ce fluorimètre conventionnel comprend une source lumineuse polychromatique (lampe Xénon 150 W), des monochromateurs à réseau, un détecteur classique et un dispositif de lecture et/ou d'enregistrement du photocourant induit. Il ne comporte en outre aucun dispositif particulier de traitement du signal. Il est clair que cette concentration minimale détectable dépend fortement des conditions d'analyse.

En conséquence, les valeurs indiquées dans les exemples ci-après, déterminées dans des conditions standards, doivent être considérées comme indicatrices des performances minimales des latex fluorescents analysés.

Pour ce faire, on réalise, à des longueurs d'ondes d'excitation et d'émission précises et pour des paramètres optiques ou électroniques du fluorimètre préalablement définis et fixés, une étude de l'intensité émise en fonction de la dilution du latex de départ.

La valeur de la concentration minimale est déduite à la lecture de la courbe correspondante d'intensité/concentration.

B - Détermination du taux d'occupation d'une particules en fluorochromes

Ce taux est accessible par dissolution d'un poids connu de latex sec dans un solvant commun au polymère et au fluorochrome. En général, il s'agit du toluène. La concentration en fluorochrome est alors déterminée par absorption UV-visible. La concentration en particules est déduite par calcul à partir du poids de polymère sec, de la taille des particules et de la masse volumique du polymère.

Le taux d'occupation $\eta p$ est alors donné par le rapport :

$$\eta p = \frac{\text{concentration en fluorochrome}}{\text{concentration en particules}}$$

EXEMPLE 1

Préparation d'un latex fluorescent marqué au 9,10 DPA et au 9,10 BPEA

Ce latex fluorescent est préparé selon la technique de gonflement en utilisant les réactifs suivants :

Latex polystyrène carboxylé de diamètre égal à 0,3 μm (123 g à 8,13 % d'extrait sec massique)
Toluène          15 g
Acétone          196 g
Laurate de potassium          0,1 g
9,10 DPA          0,028 g
9,10 BPEA          0,032 g
NH$_4$OH à 20 % qs pour pH = 10.

Le 9,10 DPA est dissous dans 7,5 g de toluène, puis additionné d'acétone (98 g). Le pH du latex est ajusté par NH$_4$OH à une valeur de 10. Le laurate de potassium est ensuite additionné au latex et l'ensemble est homogénéisé quelques minutes sous agitation. On ajoute ensuite lentement la solution de fluorochrome au latex. La suspension résultante est agitée pendant 3 heures à 40°C. Les solvants organiques sont ensuite éliminés par distillation le plus lentement possible. De l'eau désionisée est ajoutée pendant la distillation de façon à ajuster l'extrait sec aux environs de 5 %.

Le 9,10 BPEA est dissous dans le toluène restant, puis additionné d'acétone (98g g). Le pH du latex est ensuite réajusté par NH$_4$OH à une valeur de 10. On ajoute ensuite lentement la solution de fluorochrome au latex. La suspension résultante est agitée pendant 3 heures à 40°C. Les solvants organiques sont ensuite éliminés comme précédemment. L'extrait sec pondéral (ES) final est proche de 5 %. Le latex fluorescent ainsi obtenu est lavé par ultrafiltration.

Le latex obtenu présente les caractéristiques suivantes :

9,10 DPA (donneur) : 84 000 labels / particule soit 0,25 % poids polymère. Rendement d'insertion: 83 %.
9,10 BPEA (accepteur) : 123 000 labels / particule soit 0,3 % poids polymère. Rendement d'insertion : 100 %.

Le rapport de marquage accepteur/donneur est de 1,5.

On a ensuite mesuré la fluorescence photoinduite en utilisant le dispositif expérimental comprenant une source lumineuse polychromatique (lampe Xénon 150 W), des monochromateurs à réseau, un détecteur classique et un dispositif de lecture et/ou d'enregistrement du signal.

Excité à 360 nm (zone où l'absorption du 9,10 DPA est 8 fois supérieure à celle du 9,10 BPEA), le spectre d'émission du latex est pratiquement réduit à l'émission du 9,10 BPEA (Max : 480 et 510 nm). L'émission du 9,10 DPA (Max 410 et 430 nm) est quasiment inexistante (1/20ème de celle du 9,10 BPEA).

L'excitation a été enregistrée pour l'émission à 550 nm (où la fluorescence du 9,10 DPA est négligeable). Le spectre montre des épaulements distincts correspondant aux pics caractéristiques de l'excitation du 9,10 DPA.

On chiffre ensuite pour trois latex différents (9,10 BPEA, seul, 9,1() BPEA et 9,10 DPA séparés, 9,10 BPEA et 9,10 DPA dans la même particule), le rapport de l'intensité I émise à 515 nm (où l'émission du 9,10 BPEA est prépondérante) pour des excitations à 440 nm (excitation du 9,10 BPEA seul) et à 360 et 375 nm (excitation simultanée du 9,10 DPA et du 9,10 BPEA). Dans ces conditions, un transfert d'énergie du 9,10 DPA vers le 9,10 BPEA doit se traduire par une augmentation sensible des rapports I(360)/I(440) et I(375)/I(440).

On observe les résultats suivants :

a) Latex 9,10 BPEA seul : I(360)/I(440) = 0,06
      I(375)/I(440) = 0,12
b) 9,10 BPEA et 9,10 DPA dans particules séparées (mélange de latex)
      I(360)/I(440) = 0,07
      I(375)/I(440) = 0,13.

Les résultats sont équivalents à a). Aucune interaction n'est observée entre 9,10 DPA et 9,10 BPEA.
      c) 9,10 BPEA et 9,10 DPA dans la même particule.
      I(360)/I(440) = 0,22
      I(375)/I(440) = 0,36
Il y a donc bien, dans ce dernier cas, une augmentation d'un facteur 3 de l'émission du 9,10 BPEA, excité dans la zone d'absorption du 9,1() DPA lorsque les deux labels sont localisés dans la même particule.

Ceci est donc la preuve effective d'un transfert d'énergie du 9,10 DPA vers le 9,10 BPEA.

Dans ces conditions (excitation à 375 nm et émission à 480 nm), le seuil de détection de fluorescence de l'échantillon est égal à $5.10^{-15}$ moles de particules par litre.

EXEMPLE 2

Préparation d'un latex fluorescent marqué au 1,8 $Cl_2$-9,10 BPEA et au 5,12-BPEN

Le latex fluorescent est préparé selon l'exemple 1 en utilisant les réactifs suivants :
Latex selon l'exemple 1

1,8 $Cl_2$-9,10 BPEA      0,028 g
5,12-BPEN      0,032 g
Toluène      10,25 g
Acétone      196 g
Laurate de potassium      0,1 g
$NH_4OH$ à 20 %.... q.s.... pour pH = 10

Le latex obtenu présente les caractéristiques suivantes :

1,8 $Cl_2$-9,10 BPEA      77 000 labels/particule soit 0,29 % du poids du polymère
5,12-BPEN      88 000 labels/particule soit 0,32 % du poids du polymère.

Le rapport de marquage accepteur/donneur est de 1,15.
Quelle que soit la longueur d'ordre d'excitation entre 470 et 515 nm, l'émission du latex se réduit uniquement à celle du BPEN (570 nm).
On chiffre ensuite le rapport de l'intensité émise à 620 nm (émission du BPEN) pour des excitations à:

550 nm (absorption du BPEN seul)
470 nm (absorption simultanée du 5,12-BPEN et du 1,8 $Cl_2$-9,10 BPEA)
440 nm (absorption dominante du 1,8 $Cl_2$-9,10 BPEA seul).

On obtient des rapports d'intensité :

$I(470)/I(550) = 1,47$
$I(440)/I(550) = 0,52$.

Tandis que pour un latex comprenant seulement du 5,12-BPEN, les rapports d'intensité sont respectivement :

$I(470)/I(550) = 0,32$
$I(440)/I(550) = 0,08$.

On a ensuite mesuré le seuil limite de détection qui est de $6 \cdot 10^{-15}$ M de particules/L.
En conséquence, la fluorescence équivalente à une particule de latex est de 158 000 molécules de 5,12-BPEN, soit près de deux fois le marquage réel du latex en 5,12 BPEN.
Bien que le 5,12-BPEN soit le seul à émettre, cette fluorescence équivalente est très proche du nombre total de labels intéressés. L'efficacité globale de ce double marquage est de 0,96 donc comparable à celle d'un latex monomarqué en 5,12-BPEN ou au 1,8 $Cl_2$-9,10 BPEA seul.

EXEMPLE 3

Préparation d'un latex fluorescent marqué au 9,10 BPEA et au 1,8$Cl_2$-9,10 BPEA

Le latex fluorescent est préparé selon l'exemple 1 en utilisant les réactifs suivants :
Latex selon l'exemple 1

9,10 BPEA      0,028 g
1,8 $Cl_2$-9,10 BPE      0,032 g
Toluène      15 g

Acétone          196 g
Laurate de potassium          0,1 g
NH$_4$OH à 20 %.... q.s.... pour pH = 10

Le latex obtenu présente les caractéristiques suivantes :

9,10 BPEA          105 000 labels/particule soit 0,34 % du poids du polymère
1,8 Cl$_2$-9,10 BPEA          107 000 labels/particule soit 0,40 % du poids du polymère.

Le rapport de marquage accepteur/donneur est de 1,02.

Sous excitation à 400 nm (où la lumière est pratiquement absorbée par le 9,10 BPEA) l'émission est pratiquement réduite au 1,8 Cl$_2$-9,10 BPEA ($\lambda$max : 530 nm / intensité 1/2 à 565 nm).

On chiffre ensuite le rapport de l'intensité émise à 600 nm (émission du 1,8 Cl$_2$-9,10 BPEA) pour des excitations à :

500 nm (absorption du 1,8 Cl$_2$-9,10 BPEA seul)
440 nm (absorption simultanée du 9,10 BPEA et du 1,8 Cl$_2$-9,10 BPEA)
400 nm (absorption dominante du 9,10 BPEA)

On obtient des rapports d'intensité :

I(440)/I(500) = 1,4
I(400)/I(500) = 0,47

Tandis que pour un latex comprenant seulement du 1,8 Cl$_2$-9,10 BPEA, les rapports d'intensité sont respectivement :

I(440)/I(500) = 0,45
I(400)/I(500) = 0,11

On a ensuite mesuré le seuil limite de détection qui est de 2·10$^{-15}$ M de particules/L pour une excitation à 470 nm et une émission à 530 nm.

En conséquence, la fluorescence équivalente à une particule de latex est de 211 000 molécules de 1,8 Cl$_2$-9,10 BPEA, soit deux fois le marquage réel du latex au 1,8 Cl$_2$-9,10 BPEA.

Ceci équivaut au nombre total de labels insérés, d'où une efficacité globale de fluorescence égale à 1,0.

**Revendications**

1. Latex fluorescent dont les particules contiennent encapsulés au moins un fluorochrome hydrophobe A (accepteur) et au moins un fluorochrome hydrophobe D (donneur), A étant différent de D et l'émission du fluorochrome D permettant sous l'action d'un rayonnement lumineux d'exciter le fluorochrome A.

2. Latex fluorescent selon la revendication 1, caractérisé en ce que l'émission du fluorochrome D permet directement d'exciter le fluorochrome A, le spectre d'émission du fluorochrome D recouvrant partiellement le spectre d'excitation du fluorochrome A.

3. Latex fluorescent selon la revendication 2, caractérisé en ce que le rapport molaire A:D est pour au moins 10 % en poids des particules supérieur à 1.

4. Latex fluorescent selon la revendication 3, caractérisé en ce que le rapport molaire A:D est supérieur à 1, de préférence à 1,2.

5. Latex fluorescent selon l'une des revendications 2 à 4, caractérisé en ce que les fluorochromes A et D sont choisis parmi les polyaromatiques condensés, éventuellement substitués, la tétraphénylporphyrine et/ou un des complexes organométalliques de celle-ci.

6. Latex fluorescent selon la revendication 5, caractérisé en ce que les fluorochromes A et D sont choisis parmi les 9,10 diphényl-anthracène (9,10 DPA), 9,10 bis-phényl-éthynyl-anthracène (9,10 BPEA), 1,8 dichloro-9,10 bis-phényl-éthynyl-anthracène (1,8 Cl$_2$-9,10 BPEA), 5-12 bis-phényléthynyl-naphtacène (5,12 BPEN) 6,13-bis (phényléthynyl)pentacène, tétrabenzo(de,hi,op,st)pentacène, la Coumarine 153, le Rouge de Nil, le le 1,4-Di-[2(5-phényloxazolyl)] benzène ou POPOP, le 1,4-Di-[(2,-(4-méthyl-5-phényloxazolyl)] benzène ou DM-POPOP, la tetraphénylporphyrine ou TPP.

**7.** Latex fluorescent selon la revendication 6, caractérisé en ce que les fluorochromes A et D sont choisis parmi les couples suivants :

| D | A |
|---|---|
| 9,10 DPA | 9,10 BPEA |
| 1,8 $Cl_2$-9,10 BPEA | 5,12-BPEN |
| 1,8 $Cl_2$-9,10 BPEA | TPP |
| Coumarine 153 | 1,8 $Cl_2$-9,10 BPEA |
| 9,10 DPA | Coumarine 153 |
| | |
| 1,8 $Cl_2$-9,10 BPEA | Rouge de Nil |
| 9,10 BPEA | Rouge de Nil |
| POPOP | Coumarine 153 |
| 9,10 BPEA | TPP |

**8.** Latex fluorescent selon la revendication 1, caractérisé en ce que l'émission du fluorochrome D permet d'exciter indirectement le fluorochrome A, les particules du latex contenant au moins un fluorochrome intermédiaire I dont le spectre d'excitation recouvre partiellement le spectre d'émission de D et le spectre d'émission recouvre partiellement le spectre d'excitation de A.

**9.** Latex fluorescent selon la revendication 8, caractérisé en ce que les rapports des concentrations molaires A : I et I : D sont supérieures à 1, de préférence à 1,2.

**10.** Latex fluorescent selon l'une des revendications 8 ou 9, caractérisé en ce que les fluorochromes sont choisis parmi les trios suivants :

| D | I | A |
|---|---|---|
| POPOP | Coumarine 153 | 1,8 $Cl_2$-9,10 BPEA |
| 9,10 DPA | 9,10 BPEA | Rouge de Nil |
| Coumarine 153 | 1,8 $Cl_2$-9,10 BPEA | TPP |
| Coumarine 153 | 1,8 $Cl_2$-9,10 BPEA | 5,12-BPEN |

**11.** Latex fluorescent selon l'une des revendications 8 à 10, caractérisé en ce que le spectre d'émission des particules lorsqu'elles sont soumises à une excitation au moyen d'un rayonnement lumineux ne recouvre pas le spectre d'excitation desdites particules.

**12.** Latex fluorescent selon l'une des revendications 1 à 11, caractérisé en ce que le seuil de détection des particules lorsque celles-ci sont soumises à un rayonnement lumineux est inférieur ou égale à $10^{-12}$ mole de particules par litre.

**13.** Latex fluorescent selon l'une quelconque des revendications précédentes, caractérisé en ce que la taille de ses particules est comprise entre 0,01 microns et 20 microns.

**14.** Latex fluorescent selon l'une quelconque des revendications précédentes, caractérisé en ce que les particules constituent entre 0,05 et 10 % de son poids.

15. Latex fluorescent selon l'une quelconque des revendications précédentes, caractérisé en ce que le polymère constituant ses particules est un homopolymère ou copolymère contenant des motifs dérivés de monomères vinylaromatiques, éthyléniques, d'acides ou d'esters alcanoïques ou éthyléniques éventuellement fonctionnalisés.

16. Latex fluorescent selon l'une quelconque des revendications précédentes, caractérisé en ce qu'il est fonctionnalisé par au moins une espèce immunoréactive.

17. Application d'un latex fluorescent selon la revendication 16 à titre de réactif dans les tests immunologiques, en immuno-fluorescence inverse, comme scintillateur, comme standard de calibration en cytofluorométrie de flux et/ou de marqueurs cellulaires.

18. Procédé de préparation d'un latex selon l'une des revendications précédentes, caractérisé par l'une et/ou l'autre des étapes suivantes :

- introduction, en cours de polymérisation du ou des monomère(s) destinés à constituer les particules de latex, d'un ou plusieurs des fluorochromes D, A et éventuellement I en suspension dans une fraction du ou d'un des monomère(s),
- mélange d'un ou plusieurs des fluorochromes D, A et éventuellement I, solubilisés dans un solvant non aqueux, avec une dispersion aqueuse de particules de latex.

**Patentansprüche**

1. Fluoreszierender Latex, dessen Teilchen eingekapselt wenigstens ein hydrophobes Fluorochrom A (Akzeptor) und wenigstens ein hydrophobes Fluorochrom D (Donor) enthalten, wobei sich A von D unterscheidet und die Emission von Fluorochrom D durch eine Ausstrahlung von Licht die Anregung von Fluorochrom A erlaubt.

2. Fluoreszierender Latex nach Anspruch 1, dadurch gekennzeichnet, daß die Emission von Fluorochrom D eine direkte Anregung von Fluorochrom A ermöglicht, wobei das Emissionsspektrum von Fluorochrom D sich teilweise mit dem Anregungsspektrum von Fluorochrom A deckt.

3. Fluoreszierender Latex nach Anspruch 2, dadurch gekennzeichnet, daß das Molverhältnis A:D für wenigstens 10 Gew.-% der Teilchen größer ist als 1.

4. Fluoreszierender Latex nach Anspruch 3, dadurch gekennzeichnet, daß das Molverhältnis A:D größer als 1, vorzugsweise größer als 1,2 ist.

5. Fluoreszierender Latex nach einem der Ansprüche 2 bis 4, dadurch gekennzeichnet, daß die Fluorochrome A und D ausgewählt sind unter kondensierten Polyaromaten, die gegebenenfalls substituiert sind, Tetraphenylporphyrin und/oder unter einem der metallorganischen Komplexe davon.

6. Fluoreszierender Latex nach Anspruch 5, dadurch gekennzeichnet, daß die Fluorochrome A und D ausgewählt sind unter 9,10-Diphenylanthracen (9,10 DPA), 9,10-Bis-Phenylethinyl-anthracen (9,10 BPEA), 1,8-Dichlor-9,10-bis-phenyl-ethinyl-anthracen (1,8 Cl$_2$-9,10 BPEA), 5,12-Bis-phenyl-ethinyl-naphthacen (5,12 BPEN), 6,13-Bis-(phenylethinyl)-pentacen, Tetrabenzo(de,hi,op,st)-pentacen, Cumarin 153, Nilrot, 1,4-Di-[2-(5-phenyloxazolyl)]-benzol oder POPOP, 1,4-Di-[(2-(methyl-5-phenyloxazolyl)]-benzol oder DM-POPOP, Tetraphenylporphyrin oder TPP.

7. Fluoreszierender Latex nach Anspruch 6, dadurch gekennzeichnet, daß die Fluorochrome A und D unter den folgenden Paaren ausgewählt sind:

| D | A |
|---|---|
| 9,10 DPA | 9,10 BPEA |
| 1,8 Cl$_2$-9,10 BPEA | 5,12 BPEN |

(fortgesetzt)

| D | A |
|---|---|
| 1,8 Cl$_2$-9,10 BPEA | TPP |
| Cumarin 153 | 1,8 Cl$_2$-9,10 BPEA |
| 9,10 DPA | Cumarin 153 |
| 1,8 Cl$_2$-9,10 BPEA | Nilrot |
| 9,10 BPEA | Nilrot |
| POPOP | Cumarin 153 |
| 9,10 BPEA | TPP |

8. Fluoreszierender Latex nach Anspruch 1, dadurch gekennzeichnet, daß die Emission von Fluorochrom D die indirekte Anregung von Fluorochrom A ermöglicht, wobei die Latexteilchen wenigstens ein intermediäres Fluorochrom I enthalten, dessen Anregungsspektrum sich teilweise mit dem Emissionsspektrum von D deckt und dessen Emissionsspektrum sich teilweise mit dem Anregungsspektrum von A deckt.

9. Fluoreszierender Latex nach Anspruch 8, dadurch gekennzeichnet, daß die Verhältnisse der molaren Konzentrationen A:I und I:D größer als 1, vorzugsweise größer als 1,2 sind.

10. Fluoreszierender Latex nach einem der Ansprüche 8 oder 9, dadurch gekennzeichnet, daß die Fluorochrome unter den folgenden Dreiergruppen ausgewählt sind:

| D | I | A |
|---|---|---|
| POPOP | Cumarin 153 | 1,8 Cl$_2$-9,10 BPEA |
| 9,10 DPA | 9,10 BPEA | Nilrot |
| Cumarin 153 | 1,8 Cl$_2$-9,10 BPEA | TPP |
| Cumarin 153 | 1,8 Cl$_2$-9,10 BPEA | 5,12-BPEN |

11. Fluoreszierender Latex nach einem der Ansprüche 8 bis 10, dadurch gekennzeichnet, daß sich das Emissionsspektrum der Teilchen, wenn sie einer Anregung mittels Lichtausstrahlung unterworfen sind, nicht mit dem Anregungsspektrum der Teilchen deckt.

12. Fluoreszierender Latex nach einem der Ansprüche 1 bis 11, dadurch gekennzeichnet, daß die Nachweisgrenze der Teilchen, wenn diese einer Lichtausstrahlung unterworfen sind, kleiner oder gleich 10$^{-12}$ mol Teilchen pro Liter ist.

13. Fluoreszierender Latex nach irgendeinem der vorhergehenden Ansprüche, dadurch gekennzeichnet, daß die Größe seiner Teilchen zwischen 0,01 µm und 20 µm liegt.

14. Fluoreszierender Latex nach irgendeinem der vorhergehenden Ansprüche, dadurch gekennzeichnet, daß die Teilchen zwischen 0,05 und 10 % seines Gewichtes ausmachen.

15. Fluoreszierender Latex nach irgendeinem der vorhergehenden Ansprüche, dadurch gekennzeichnet, daß das Polymer, aus dem seine Teilchen bestehen, ein Homopolymer oder Copolymer ist, das Einheiten enthält, die sich von vinylaromatischen, ethylenischen, Säure- oder Alkanester- oder ethylenischen, gegebenenfalls funktionalisierten, Monomeren ableiten.

16. Fluoreszierender Latex nach irgendeinem der vorhergehenden Ansprüche, dadurch gekennzeichnet, daß er mit wenigstens einer immunreaktiven Spezies funktionalisiert ist.

17. Anwendung eines fluoreszierenden Latex nach Anspruch 16 als Reagens bei immunologischen Tests, bei der inversen Immunfluoreszenz, als Szintillator, als Eichstandard in der Cytofluorometrie für den zellulären Fluß und/ oder die Zellmarker.

18. Verfahren zur Herstellung eines Latex nach einem der vorhergehenden Ansprüche, gekennzeichnet durch den einen und/oder anderen der folgenden Schritte:

- Einbringen während des Ablaufes der Polymerisation des oder der Monomeren, die zum Aufbau der Latexteilchen bestimmt sind, eines oder mehrerer der Fluorochrome D, A und gegebenenfalls I in Suspension in eine Fraktion der oder des Monomeren,
- Mischen eines oder mehrerer der Fluorochrome D, A und gegebenenfalls I, die in einem nichtwäßrigen Lösungsmittel gelöst sind, mit einer wäßrigen Dispersion der Latexteilchen.

## Claims

1. Fluorescent latex the particles of which contain at least one hydrophobic fluorochrome A (acceptor) and at least one hydrophobic fluorochrome D (donor) which are encapsulated, A being different from D and the emission of the fluorochrome D making it possible, under the action of light radiation, to excite the fluorochrome A.

2. Fluorescent latex according to Claim 1, characterized in that the emission of the fluorochrome D makes it possible to excite the fluorochrome A directly, the emission spectrum of the fluorochrome D partially overlapping the excitation spectrum of the fluorochrome A.

3. Fluorescent latex according to Claim 2, characterized in that for at least 10% by weight of the particles, the A:D molar ratio is greater than 1.

4. Fluorescent latex according to Claim 3, characterized in that the A:D molar ratio is greater than 1, preferably greater than 1.2.

5. Fluorescent latex according to one of Claims 2 to 4, characterized in that the fluorochromes A and D are chosen from condensed, optionally substituted polyaromatics, tetraphenylporphyrine and/or one of the organometallic complexes thereof.

6. Fluorescent latex according to Claim 5, characterized in that the fluorochromes A and D are chosen from 9,10 diphenyl-anthracene (9,10 DPA), 9,10 bis-phenylethynyl-anthracene (9,10 BPEA), 1,8 dichloro 9,10 bisphenyl-ethynyl-anthracene (1,8 $Cl_2$-9,10 BPEA), 5-12 bis-phenyl-ethynyl-naphtacene (5,12 BPEN), 6,13-bis (phenylethynyl)pentacene, tetrabenzo(de,hi,op,st)pentacene, Coumarin 153, Nile red, 1,4-di[2(5-phenyloxazolyl)]benzene or POPOP, 1,4-di[(2-(4-methyl-5-phenyloxazolyl)]benzene or DM-POPOP, and tetraphenylporphyrine or TPP.

7. Fluorescent latex according to Claim 6, characterized in that the fluorochromes A and D are chosen from the following pairs:

| D | A |
|---|---|
| 9,10 DPA | 9,10 BPEA |
| 1,8 $Cl_2$-9,10 BPEA | 5,12 BPEN |
| 1,8 $Cl_2$-9,10 BPEA | TPP |
| Coumarin 153 | 1,8 $Cl_2$-9,10 BPEA |
| 9,10 DPA | Coumarin 153 |
| 1,8 $Cl_2$-9,10 BPEA | Nile red |
| 9,10 BPEA | Nile red |
| POPOP | Coumarin 153 |
| 9,10 BPEA | TPP |

8. Fluorescent latex according to Claim 1, characterized in that the emission of the fluorochrome D makes it possible to excite the fluorochrome A indirectly, the particles of the latex containing at least one intermediate fluorochrome I whose excitation spectrum partially overlaps the emission spectrum of D and the emission spectrum partially overlaps the excitation spectrum of A.

9. Fluorescent latex according to Claim 8, characterized in that the molar concentration ratios A:I and I:D are greater than 1, preferably greater than 1.2.

10. Fluorescent latex according to either of Claims 8 and 9, characterized in that the fluorochromes are chosen from the following trios:

| D | I | A |
|---|---|---|
| POPOP | Coumarin 153 | 1,8 $Cl_2$-9,10 BPEA |
| 9,10 DPA | 9,10 BPEA | Nile red |
| Coumarin 153 | 1,8 $Cl_2$-9,10 BPEA | TPP |
| Coumarin 153 | 1,8 $Cl_2$-9,10 BPEA | 5,12-BPEN |

11. Fluorescent latex according to one of Claims 8 to 10, characterized in that when they are subjected to an excitation using a light radiation, the emission spectrum of the particles does not overlap the excitation spectrum of the said particles.

12. Fluorescent latex according to one of Claims 1 to 11, characterized in that when they are subjected to a light radiation, the detection threshold of the particles is less than or equal to $10^{-12}$ mol of particles per litre.

13. Fluorescent latex according to any one of the preceding claims, characterized in that its particles are between 0.01 micron and 20 microns in size.

14. Fluorescent latex according to any one of the preceding claims, characterized in that the particles constitute between 0.05 and 10% of its weight.

15. Fluorescent latex according to any one of the preceding claims, characterized in that the polymer constituting its particles is a homopolymer or copolymer containing units derived from vinylaromatic or ethylenic monomers or from alkanoic or ethylenic acids or esters, which are optionally functionalized.

16. Fluorescent latex according to any one of the preceding claims, characterized in that it is functionalized with at least one immunoreactive species.

17. Application of a fluorescent latex according to Claim 16 as a reagent in immunological tests, in reverse immunofluorescence, as a scintillator, as a calibration standard in flow cytofluorimetry and/or as cell markers.

18. Process for the preparation of a latex according to one of the preceding claims, characterized by one and/or other of the following steps:

- introduction, during the polymerization of the monomer(s) intended to constitute the latex particles, of one or more of the fluorochromes D, A and optionally I in suspension in a fraction of the monomer or of one of the monomers,
- mixing of one or more of the fluorochromes D, A and optionally I, solubilized in a non-aqueous solvent, with an aqueous dispersion of latex particles.